# EUROPÄISCHE PATENTANMELDUNG

(11) **EP 3 922 278 A1**
(43) Veröffentlichungstag der Anmeldung: **15.12.2021**
(21) Anmeldenummer: 20209035.3
(22) Anmeldetag: 20.11.2020
(51) Int. Cl.: A61L 9/20, F21V 7/00

(54) **LEUCHTE UND SYSTEM MIT WANDARTIGEN STRAHLUNGSFELDERN ZUR VERHINDERUNG ODER MINIMIERUNG DER VERBREITUNG VON KRANKHEITSERREGERN IN RAUMLUFT**

(30) Priorität: 11.06.2020 EP 20179592; 26.08.2020 DE 102020122343; 29.09.2020 DE 102020125384
(71) Anmelder: Smart United GmbH, 82031 Grünwald (DE)
(72) Erfinder: PROHASKA, Reiner, 82031 Grünwald (DE); WIESER, Andreas, 80687 München (DE)
(74) Vertreter: Beder, Jens

(57) **Zusammenfassung**

Die Erfindung geht aus von einem System um eine Verbreitung von Viren zu verhindern oder zu minimieren und zur Verhinderung oder Minimierung der Verbreitung von Viren in Raumluft mit einer oder mehreren Strahlungsquellen (10) in einem Raum, die die Räume durch sogenannte UV-C Lichtwände den Raum in kleinere Segmente unterteilt, einer Sensoranordnung zum Detektieren einer Bewegung oder einer Anwesenheit einer oder mehrerer Personen (P) in dem Raum und einer Steuerung (16), die dazu ausgelegt ist, die eine oder mehreren Strahlungsquellen (10) abhängig von wenigstens der Anwesenheit der Person (P) zumindest teilweise ein- oder auszuschalten.

Es wird vorgeschlagen, dass die eine oder die mehreren Strahlungsquellen (10) dazu ausgelegt sind, jeweils ein wandartiges Strahlungsfeld (10b) zu erzeugen, welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden, und die Steuerung (16) dazu ausgelegt ist, die betreffende Strahlungsquelle (10) zumindest teilweise abzuschalten, wenn die aus den von der Sensoranordnung detektierten Bewegungsdaten es wahrscheinlich erscheinen lassen, dass eine der Personen (P) das betreffende Strahlungsfeld (10b) durchqueren möchte.

## Beschreibung

Die Erfindung betrifft eine Leuchte und ein System mit wandartigen Strahlungsfeldern zur Desinfektion von Raumluft und um die Verbreitung von Krankheitserregern, insbesondere Viren, in Gebäuden zu verhindern/minimieren sowie ein entsprechendes Verfahren.

Nicht erst seit der weltweiten Corona-Pandemie ist die gesundheitliche Bedeutung Hygienemaßnahmen in Räumen bekannt, insbesondere dort, wo eine Vielzahl von Personen zusammenarbeitet und/oder ein- und ausgeht.

Dabei wurden bereits verschiedene Systeme zur Desinfektion von Raumluftmit UV-Strahlung vorgeschlagen.

Zweckmäßige keimtötende Strahlung ist UV-Strahlung. Eine geeignete Strahlungsquelle ist daher eine UV-Lampe, die allgemein UV-Strahlung abgibt, die etwa im Wellenlängenbereich von 100 bis 400 nm liegt. Innerhalb der UV-Strahlung nimmt die keimtötende Wirkung von UV-A über UV-B bis UV-C mit kürzerer Wellenlänge zu. Besonders geeignet ist daher eine UV-C- Lampe, die speziell UV-C-Strahlung abgibt, die etwa im Wellenlängenbereich von 100 bis 280 nm liegt. Bevorzugt ist der Wellenlängenbereich etwa von 200 bis 280 nm, da Luft in diesem Bereich im Wesentlichen transparent für die Strahlung ist. Bekannte Strahlungsquellen dieser Art sind Quecksilberdampf-Lampen oder Leuchtdioden oder Laserdioden zur Abstrahlung entsprechenden UV-Lichts.

Keimtötende UV-C Strahlung kann schädlich für die menschlichen Augen und Haut sein. Maßnahmen, um daher Personen vor der Exposition mit der keimtötenden UV-C Strahlung zu schützen, können einen Spiegel, eine Blende und/oder einen Schirm zum Bündeln, Richten und Begrenzen der Strahlung umfassen. Vorzugsweise umfassen sie einen Sensor zur Erfassung der Anwesenheit einer Person im räumlichen Bereich, in dem die Strahlung wirken kann, insbesondere zur Erfassung der Anwesenheit einer Person im Bereich unmittelbar vor oder neben der Strahlungsquelle. Ein Schalter ist mit dem Sensor und der Strahlungsquelle gekoppelt und schaltet diese ab, wenn der Sensor die Person erfasst.

In dem Paper Welch, D., Buonanno, M., Grilj, V. et al. "Far-UV-C light: A new tool to control the spread of airborne-mediated microbial diseases" Sci Rep 8, 2752 (2018); wird allerdings beschrieben dass sehr kurzwelliges UV-C-Licht (207-222 nm), auch als Far-UV-C-Licht bezeichnet, effizient Bakterien inaktiviert, ohne die exponierte Haut von Säugetieren zu schädigen. Der Grund dafür ist, dass Far-UV-C-Licht aufgrund seiner starken Absorption in biologischen Materialien die äußeren (nicht lebenden) Schichten der menschlichen Haut oder des Auges nicht durchdringen kann. Da Bakterien und Viren jedoch Dimensionen haben von einem Mikrometer oder weniger habe, kann Far-UV-C-Licht in sie eindringen und inaktivieren. Es wird gezeigt, dass Far-UV-C aerosolisierte Viren in der Luft effizient inaktiviert, wobei eine sehr geringe Dosis von 2 mJ/cm² von 222-nm-Licht mehr als 95% eines aerosolisierten H1N1-Grip- pevirus inaktiviert.

Geeignete Sensoren zum Erfassen der Anwesenheit von Personen sind Bewegungsmelder wie beispielsweise Ultraschall- oder Radarsensoren, die den Dopplereffekt bei Reflektion der von ihnen abgegebenen Ultraschall- oder Radarstrahlung an einer sich bewegenden Person nutzen, oder passive pyroelektrische IR-Sensoren (PIR-Sensoren), die die von einer sich bewegenden Person verursachten Änderungen der Wärmestrahlung in der Umgebung des Möbels erfassen. Geeignet sind auch Näherungssensoren wie beispielsweise kapazitive, optische, Ultraschall- oder Radarsensoren, die eine Person in der Nähe unabhängig von ihrer Bewegung erfassen können.

Aus der WO 2016049143 A1 ist beispielsweise ein System zum Dekontaminieren von Nasszellen in Krankenhäusern bekannt. In der Nasszelle ist eine UV-C-Lichtquelle angeordnet, die abgeschaltet wird, sobald eine Person den Raum betritt.

Aus der US 9,550,006 B2 ist ein System mit UV-Strahlungsquellen zur Installation in Passagierkabinen von Flugzeugen bekannt. Ein Sicherheitssystem aktiviert oder deaktiviert die Strahlungsquellen, wenn ein Passagier o- der Crew-Mitglied die Passagierkabine betritt.

Aus der US 9, 095, 633 B1 ist ein mobiles System zur Dekontamination von Krankenzimmern bekannt. Dieses System wird in dem Krankenzimmer aufgestellt und über einen Zeitschalter gestartet, wenn alle Personen den Raum verlassen haben.

Aus der WO 2015 054389 A2 sind UV-undurchlässige Strahlenschutzvor- hänge bekannt, mit denen bestimmte zu desinfizierende Bereiche eines Raums (z.B. ein Bett eines Mehrbett-Krankenzimmers) abgetrennt werden können, um eine Desinfektion des abgetrennten Bereichs mit UV-Strahlungsquellen zu ermöglichen, während sich Personen in anderen Bereichen des Raums aufhalten können. Aus der WO 2014 100493 A1 ist ein ähnliches System zum gleichen Zweck mit Stellwänden bekannt, an deren Innenseite UV-Strahlungsquellen angeordnet sind.

Eine mobile UV-Strahlungsquelle, die in zu desinfizierenden Räumen oder mit UV-Schutz abgetrennten Raumbereichen aufgestellt werden kann, ist beispielsweise aus der WO 2012142427 A1 oder der US 6,656,424 B1 bekannt.

Ein Nachteil der oben genannten Systeme ist, dass der zu dekontaminierende Raum entweder vollständig menschenleer sein muss oder ein aufwändiger Aufbau von Strahlenschutzwänden oder Vorhängen erforderlich ist. In Räumen mit häufigem und unvorhersehbarem Publikumsverkehr ist dies nicht realisierbar.

Zur kontinuierlichen Desinfektion von Raumluft in Räumen, die dauerhaft belegt sind, beispielswiese Wartezimmern von Arztpraxen, ist es bekannt, UV-C-Strahlungsquellen innerhalb von Gehäusen von Lüftern, Klimaanlagen oder Ventilatoren anzuordnen. Aus der US 2009 004046 A1 ist beispielsweise eine derartige Vorrichtung zur Deckenmontage bekannt. Ein Nachteil dieser im Umluftverfahren arbeitenden Systeme ist, dass eine Diffusion von eventuell infektiösen Aerosolen zwischen Personen, die sich in dem betreffenden Raum befinden, möglich bleibt und die Aerosole sich durch den Luftzug der Umluft unter Umständen sogar noch schneller ausbreiten, als dies ohne die Umluft-System der Fall wäre.

Der Erfindung liegt die Aufgabe zugrunde, eine Leuchte sowie ein System mit durch ein oder mehrere solcher Leuchten erzeugten wandartigen Strahlungsfeldern zur Verhinderung oder Minimierung der Verbreitung von Krankheitserregern in Raumluft bereitzustellen, die bei Positionierung der Strahlungsfelder zwischen Personen eine Übertragung von Keimen zwischen diesen Personen wirkungsvoll verhindert, ohne die Bewegungsfreiheit der Personen zu beeinträchtigen.

Die Aufgabe wird gelöst durch eine Leuchte zur Ausbildung einer Barriere in Form eines wandartigen Strahlungsfelds mit den Merkmalen des Anspruchs 1 sowie ein System mit solchen wandartigen Strahlungsfeldern zur Verhinderung oder Minimierung der Verbreitung von Krankheitserregern, insbesondere Viren, in Raumluft. Vorteilhafte Ausgestaltungen der Erfindung ergeben sich aus den Unteransprüchen. "Barriere" im Sinne der vorliegenden Ausführungen ist hier nicht im Sinne einer mechanischen Begrenzung zu verstehen. Vielmehr bedeutet Barriere, dass Krankheitserreger, insbesondere Viren, zwar auf die andere Seite der Barriere gelangen können, beim Durchtritt durch die Barriere aber deaktiviert werden.

Die Erfindung betrifft eine Leuchte sowie ein System zur Verhinderung oder Minimierung der Verbreitung von Krankheitserregern in Raumluft mit einer oder mehreren solcher Leuchten als Strahlungsquellen in einem Raum oder mehreren Räumen, insbesondere mit einer Sensoranordnung zum Detektieren einer Bewegung oder einer Anwesenheit einer oder mehrerer Personen in dem Raum und einer Steuerung, die dazu ausgelegt ist, die eine oder mehreren Strahlungsquellen abhängig von wenigstens der Anwesenheit der Person ein- oder auszuschalten.

Zum Ausbilden der Barriere für Krankheitserreger weist die Leuchte zum Erzielen der keimtötenden Wirkung wenigstens ein UV-C Strahlung emittierendes Leuchtmittel auf. Nachfolgend wird zur Vereinfachung meist lediglich auf Viren als Beispiel Bezug genommen, obwohl die erfindungsgemäß ausgebildete Barriere auch in Bezug auf Bakterien ihre Wirkung hat. Die von dem einen bzw. den mehreren Leuchtmitteln emittierte Strahlung wird mit Hilfe einer optischen Einrichtung kollimiert, so dass ein Strahlungsfeld entsteht, dessen Dicke wenigstens eine Größenordnung kleiner als seine Länge und Breite ist, also lediglich maximal 1/10 der Länge oder Breite.

Die optische Einrichtung ist also so beschaffen, dass die von dem Leuchtmittel emittierte Strahlung im Wesentlichen nur innerhalb eines Bereichs austritt, der durch zwei zueinander parallele Ebenen begrenzt ist. Der Abstand dieser Ebenen ist die oben genannte Dicke. Die "Länge" bezeichnet dabei die Dimension in Richtung der aus der Leuchte austretenden Strahlung und "Breite" eine Erstreckung senkrecht zur Länge und Dicke. Dabei ist die Länge als eine mindestens nutzbare Erstreckung des Strahlungsfelds in Abstrahlrichtung zu verstehen, die zum Beispiel bei einer Montage der Leuchte an der Decke eines Raums die Distanz bis zum Fußboden ist. Typische Dimensionen von Räumen haben dabei Höhen von bis zu 5m, sodass bevorzugt eine mindestens nutzbare Erstreckung von 5m vorzusehen ist. Es ist bevorzugt, die von dem Leuchtmittel emittierte Strahlung noch stärker zu kollimieren, sodass die Dicke bevorzugt wenigstens zwei Größenordnungen kleiner als die Mindestlänge und Breite des Strahlungsfelds ist.

Für eine Leuchte, die in einem Raum eingesetzt werden soll, ist es besonders bevorzugt, dass die Dicke, also der Abstand der parallelen Ebenen, in dem sich die kollimierte Strahlung ausbreitet, bei einer mindestens nutzbaren Erstreckung von 5 m, einen Wert von 8 cm, bevorzugt 5 cm, nicht überschreitet. typische Breiten des Strahlungsfelds, und damit auch der Leuchten können ebenfalls bis zu 5 m betragen. Bevorzugt ist es allerdings, die Leuchten weniger lang auszuführen, was Montage und Transport erheblich erleichtert. Die leuchten können dann zur Erzielung größerer Gesamtbreiten auch in einer Linie aufeinanderfolgend angeordnet werden.

Die optische Einrichtung kann dabei bevorzugt auch eine Abblendeinrichtung umfassen. Diese Abblendeinrichtung verhindert, dass Strahlungsanteile seitlich aus dem Strahlungsfeld austreten. Die Abblendeinrichtung kann beispielsweise durch eine Mehrzahl von Kanälen gebildet sein, wobei die Gesamtheit der Kanäle eine Lichtaustrittsfläche bildet oder vor der Lichtaustrittsfläche in der Leuchte angeordnet ist und sämtliche von der Leuchte emittierte Strahlung ausschließlich durch diese Gesamtheit von Kanälen austreten kann. Dabei sind die Wände der Kanäle mit die emittierte Strahlung absorbierendem Material beschichtet oder aus absorbierendem Material gefertigt. Auf diese Weise wird erreicht, dass lediglich der kollimierte Anteil der durch die Leuchtmittel emittierte Strahlung ohne Absorption durch die Kanäle hindurch austreten kann. Streulicht, dass dem nicht kollimierten Anteil der Strahlung entspricht, wird durch die Abblendeinrichtung am Austritt in die Umgebung gehindert. Die letztlich die Leuchte verlassende UV-C Strahlung kann so effizient auf den zwischen den begrenzenden, gedachten Ebenen ausgebildeten Bereich beschränkt werden. Dieser Bereich bildet eine sogenannte UV Wand.

Gemäß einer weiteren bevorzugten Ausführungsform weist die Leuchte eine Mehrzahl von UV-C Strahlung emittierenden Leuchtmitteln auf. Zudem umfasst die optische Einrichtung der Leuchte eine Mehrzahl von optischen Elementen zum Kollimieren der von den Leuchtmitteln emittierten Strahlung. Dabei ist jedem Leuchtmittel jeweils wenigstens ein optisches Element zugeordnet. Die Mehrzahl von Leuchtmitteln sowie diesen zugeordnete optische Elemente bilden dabei zumindest eine Gruppe aus. Die Abstrahlrichtungen, die für jedes Leuchtmittel durch das wenigstens eine zugeordnete optische Element entstehen, sind für sämtliche Leuchtmittel, die zu derselben Gruppe gehören, parallel zueinander und liegen in einer gemeinsamen Ebene. Damit kann eine Mehrzahl von individuellen Leuchtmitteln gemeinsam mit dem jeweils dem Leuchtmittel zugeordneten optischen Element zur Ausbildung des oben bereits erläuterten wandartigen Strahlungsfelds zusammenwirken. Bei Ausbildung von zwei oder mehr Gruppen können diese insbesondere so ausgebildet sein, dass die Abstrahlrichtungen der Leuchtmittel der einen Gruppe parallel zu den Abstrahlrichtungen der Leuchtmittel der anderen Gruppe ausgerichtet sind.

Es ist insbesondere bevorzugt, dass die optischen Elemente so ausgebildet und in der Leuchte angeordnet sind, dass die kollimierte Strahlung eines Leuchtmittels mit der kollimierten Strahlung eines benachbarten Leuchtmittels derselben Gruppe überlappt. Auf diese Weise bildet das Strahlungsfeld der Gesamtheit jeweils einer Gruppe zugeordneter Leuchtmittel gemeinsam eine lückenlose Barriere, auch als wandartiges Strahlungsfeld oder UV Wand bezeichnet, für Viren.

Jede Gruppe von Leuchtmitteln samt ihrer optischen Elemente kann auch in Untergruppen aufgeteilt sein und ein unabhängiges An- und Abschalten der Leuchtmittel verschiedener Untergruppen ermöglicht sein. Damit lässt sich zum Beispiel aus einer Gruppe, die gemeinsam eine relativ große Barriere für Viren bildet, lediglich ein kleiner Teil von Leuchtmitteln ausschalten, sofern in diesem Bereich ein Sicherheitsrisiko für eine Person entstehen könnte. Die übrigen Untergruppen können währenddessen angeschaltet bleiben. Das Abschalten nicht aller Leuchtmittel der gesamten Leuchte hat den Vorteil, dass zumindest in Teilbereichen das Strahlungsfeld aufrechterhalten werden kann, sodass zumindest teilweise Schutz bestehen bleibt. Die Granularität kann darüber bestimmt werden, in wie viele Untergruppen eine Gruppe von Leuchtmitteln unterteilt wird. Im Extremfall bildet jeweils ein Leuchtmittel eine Untergruppe aus.

Es wird außerdem vorgeschlagen, dass die eine oder die mehreren Strahlungsquellen, die durch die vorstehend erläuterten Leuchten gebildet sein können, dazu ausgelegt sind, durch gebündeltes UV-C-Licht jeweils ein wandartiges Strahlungsfeld zu erzeugen, welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden. In Varianten mit einer Sensoranordnung ist die Steuerung vorteilhaft dazu ausgelegt, die betreffende Strahlungsquelle, oder Teile davon, abzuschalten, wenn die aus den von der Sensoranordnung detektierten Bewegungsdaten zeigen, dass eine der Personen sich dem betreffenden Strahlungsfeld nähert.

Eine solche Annäherung an das Strahlungsfeld kann zum Beispiel dann angenommen werden, wenn die Sensoranordnung ein Eindringen eines Objekts in eine Sicherheitszone, die benachbart zu dem Strahlungsfeld ausgebildet ist und mit der Sensoranordnung überwacht wird, festgestellt wird. Das Eindringen eines Objekts kann dabei eine Person (bzw. lediglich ein Körperteil der Person, wie beispielsweise einen Finger) betreffen, aber auch andere Objekte. Durch das Erkennen des Eindringens von Objekten in den Sicherheitsbereich und entsprechendes (selektives) Abschalten der entsprechenden Untergruppe oder Untergruppen von Leuchtmitteln kann auch verhindert werden, dass eine mittelbare Gefährdung von Personen durch reflektierte Strahlungsanteile entsteht. Zum selektiven Abschalten einer oder mehrerer Untergruppen wird das Eindringen der Objekte in die Sicherheitszone ortsaufgelöst in mindestens eine Dimension ermittelt.

Insbesondere wenn Far-UV-C-Strahlung verwendet wird, kann aus den oben beschriebenen Gründen auch auf die Sensoranordnung und das Abschalten verzichtet werden, da gesundheitliche Gefahren nicht zu befürchten sind.

Die wandartigen Strahlungsfelder, die insbesondere UV-C-Strahlung mit hoher Intensität enthalten, bilden Diffusionsbarrieren für Keime. Die Intensität und Wellenlänge des Strahlungsfelds ist so abgestimmt, dass Keime oder Viren, die in Aerosolen oder Tröpfchen enthalten sein können, beim Durchqueren des wandartigen Strahlungsfelds abgetötet werden. Die Ansteckungswahrscheinlichkeit kann so für Personen, die sich in durch ein derartiges Strahlungsfeld voneinander getrennten Raumsegmenten aufhalten, stark reduziert werden. Auch wenn die Viren nicht vollständig abgetötet werden, kann eine Wirkung erzielt werden, die derjenigen von Mund-Nasenschutzmasken oder "Social-Distancing" - Maßnahmen entspricht oder überlegen ist.

Ein Algorithmus, der eine Wahrscheinlichkeit für ein baldiges Durchqueren des Strahlungsfelds berechnet, kann neben der Position der Person, d.h. der Nähe der Person zum Strahlungsfeld, auch die Bewegungsrichtung und Bewegungsgeschwindigkeit der Person berücksichtigen sowie gewisse Randbedingungen des Raums. Solche Randbedingungen können beispielsweise durch Möbel definiert sein, deren Standorte in der Steuerung hinter- legt sind. Unter normalen Umständen wird es sehr unwahrscheinlich sein, dass eine Person über einen Tisch oder einen Raumteiler klettert oder springt.

Als "wandartiges" Strahlungsfeld soll ein Strahlungsfeld bezeichnet werden, das eine annähernd zweidimensionale Fläche bildet, also eine Dicke hat, die wenigstens eine Größenordnung kleiner ist als ihre Länge und ihre Breite. Das wandartige Strahlungsfeld kann insbesondere auch aus mehreren, eng nebeneinander liegenden, parallel orientierten Strahlen bestehen, beispielsweise Laserstrahlen.

Die Erfindung ist auf verschiedene Räume anwendbar, in denen sich Menschen aufhalten, z.B. Großraumbüros, Klassenzimmer in Schulen, Mehrbettrankenzimmer, Restaurants oder Arbeitsplätze in der Industrie.

Wegen der oben im Zusammenhang mit dem Paper von Welch et. Al. diskutierten Vorteile sind Ausführungsformen besonders vorteilhaft, in denen die Viren deaktivierende UV-C-Strahlung eine Far-UV-C Strahlung mit einer Wellenlänge im Bereich von 200 - 222 nm, insbesondere 207 - 222 nm ist. Wegen der technisch ausgereifteren Strahlungsquellen und aus Kostengründen kann in bestimmten Anwendungsgebieten auch ein Wellenlängenbereich von 223 - 280 nm vorteilhaft sein.

Ferner wird vorgeschlagen, dass die eine oder die mehreren Strahlungsquellen als Lichtleisten zur Decken- oder Wandmontage ausgestaltet sind. Jede der Strahlungsquellen kann mit einem oder mehreren UV-C-Strahlern, beispielsweise LEDs oder Laserdioden, ausgestattet sein oder eine stärkere UV-Lichtquelle wie eine Quecksilberdampflampe oder einen gepumpten Laser umfassen deren Licht dann durch eine geeignete optische Anordnung fächerartig aufgesplittet werden kann, um die gewünschte wandartige Form zu erzeugen. Durch die Ausgestaltung als montierbare Lichtleisten ist ein flexibler Einsatz, auch beim Nachrüsten von Räumen, möglich.

Wenn das System freibewegliche Ständer zum Halten einer oder mehrerer Strahlungsquellen umfasst, ist das System auch in Bereichen einsetzbar, in denen die räumlichen Gegebenheiten eine Wand- oder Deckenmontage nicht erlauben.

In einer weiteren Ausgestaltung der Erfindung sind die eine oder die mehreren Strahlungsquellen jeweils zur Erzeugung mehrerer, parallel verlaufender Strahlungsfelder ausgelegt, so dass eine Doppelwand oder Mehrfachwand erzeugt wird. Dadurch kann die Schutzwirkung weiter verbessert werden.

Ferner wird vorgeschlagen, dass die eine oder die mehreren Strahlungsquellen zur Anordnung entlang von Begrenzungen von Raumsegmenten ausgelegt sind, wobei die Steuerung dazu ausgelegt ist, die betreffenden Strahlungsquellen zu aktivieren, wenn eine oder mehrere Personen sich in dem betreffenden Raumsegment aufhält und wenigstens eine der Strahlungsquellen zu deaktivieren, wenn eine Person das Raumsegment betritt oder verlässt.

Ferner wird vorgeschlagen, dass die Raumsegmente ein regelmäßiges Raster bilden. Dadurch können großflächige Räume flexibel abgedeckt werden.

In einer weiteren Ausgestaltung der Erfindung sind in den Raumsegmenten weitere Strahlungsquellen mit desinfizierender bzw. Viren deaktivierender Wirkung angeordnet. Die Steuerung kann dann dazu ausgelegt sein, die weiteren Strahlungsquellen zu aktivieren, wenn sich keine Person in dem Raumsegment befindet. Dadurch können Oberflächen, Computer, Stühle etc. wirkungsvoll desinfiziert werden, währen keine Person sich in dem Raumsegment aufhält.

Ferner wird vorgeschlagen, dass die Sensoranordnung eine 3D-Kamera bzw. TOF-Kamera und/oder eine oder mehrere CCD-Kameras umfasst, um die dreidimensionale Position und Pose der Personen in dem betreffenden Raumsegment detektieren zu können und auswerten zu können.

Ein weiterer Aspekt der Erfindung betrifft ein Verfahren zur Verhinderung oder Minimierung der Verbreitung von Viren in Raumluft mit einer oder mehreren Strahlungsquellen in einem Raum, optional umfassend das Detektieren einer Bewegung oder einer Anwesenheit einer oder mehrerer Personen in dem Raum und das automatische Ein- oder Ausschalten einer oder mehrerer Strahlungsquellen abhängig von wenigstens der Anwesenheit der Person.

Es wird vorgeschlagen, dass die eine oder die mehreren Strahlungsquellen dazu ausgelegt sind, jeweils ein wandartiges Strahlungsfeld zu erzeugen, , welches als UV-C-Wand wirkt, dadurch den Raum oder die Räume in kleinere Raumsegmente aufzuteilen die eine Verbreitung von Viren verhindert oder minimiert, da die Viren durch das UV-C Licht deaktiviert werden und das Verfahren das Abschalten der betreffenden Strahlungsquelle, wenn die aus den von der Sensoranordnung detektierten Bewegungsdaten es wahrscheinlich erscheinen lassen, dass eine der Personen das betreffende Strahlungsfeld durchqueren möchte, umfasst.

Die Erfindung betrifft zudem ein System um Verbreitung von Viren in Räumen zu verhindern oder zu minimieren und zur Desinfektion von Raumluft mit einer oder durch mehrere zusammen geschalteten Strahlungsquellen dadurch gekennzeichnet, dass die eine oder die mehrere zusammen geschalteten Strahlungsquellen durch gebündeltes UV-C Licht, sogenannte Lichtwände bilden und dadurch Räume in kleinere Segmente aufteilt die eine Verbreitung von Viren verhindert oder minimiert, da die Viren durch das UV-C Licht deaktiviert werden, in Kombination mit Bewegungsmeldern die einzelne UV-C Lichtwände ausschalten wenn sich eine Person nähert o- der sich wieder einschalten wenn die Person sich entfernt und einem zusätzlichen UV-C Strahler der die einzelnen durch die eine oder mehrere UV-C Lichtwände entstanden Parzelle aufgeteilten Räume ausstrahlt und die Aerosole (in der Luft vorhandenen Viren) deaktiviert.

Weitere Merkmale und Vorteile ergeben sich aus der folgenden Figurenbeschreibung. Die gesamte Beschreibung, die Ansprüche und die Figuren offenbaren Merkmale der Erfindung in speziellen Ausführungsbeispielen und Kombinationen. Der Fachmann wird die Merkmale auch einzeln betrachten und zu weiteren Kombinationen oder Unterkombinationen zusammenfassen, um die Erfindung, wie sie in den Ansprüchen definiert ist, an seine Bedürfnisse oder an spezielle Anwendungsbereiche anzupassen.

Dabei zeigen:
- Fig. 1: ein System zur Verhinderung oder Minimierung der Verbreitung von Viren in Raumluft nach einem ersten Ausführungsbeispiel der Erfindung;
- Fig. 2a - 2c: ein einzelnes Raumsegment des Systems aus Fig. 1 in drei verschiedenen Zuständen;
- Fig. 3a und 3b: eine schematische Schnittansicht einer Strahlungsquelle und eines wandartigen Strahlungsfelds gemäß zwei verschiedenen Ausführungsbeispielen der Erfindung;
- Fi. 4: ein Diagramm zum Erläutern der Bündelung von Strahlung zum Erzeugen des wandartigen Strahlungsfelds als Barriere;
- Fig. 5: eine vergrößerte Darstellung eines Ausschnitts der Figur 4 zur Erläuterung der Funktion der Abblendeinrichtung;
- Fig. 6: ein Beispiel für eine Anordnung optischer Elemente benachbarter Leuchtmittel;
- Fi. 7: eine Schnitt durch das erzeugte Strahlungsfeld mit Darstellung einer durch eine Sensoranordnung überwachten Sicherheitszone;
- Fig. 8: eine Seitenansicht zur Erläuterung des selektiven Abschaltens von Untergruppen bei erkanntem Eindringen eines Objekts in die Sicherheitszone; und
- Fig. 9: eine weitere Ausgestaltung der Erfindung mit einem Ständer für die Strahlungsquellen eines erfindungsgemäßen Systems.

Bevor auf eine konkrete Ausführungsform zur Erläuterung einer erfindungsgemäßen Leuchte zum Erreichen des sich aus der Erfindung ergebenden Schutzes von Personen in einem Raum vor Ansteckung mit über Luft übertragbaren Erregern eingegangen wird, soll zunächst das mit Hilfe der erfindungsgemäßen Leuchte errichtete System erläutert werden.

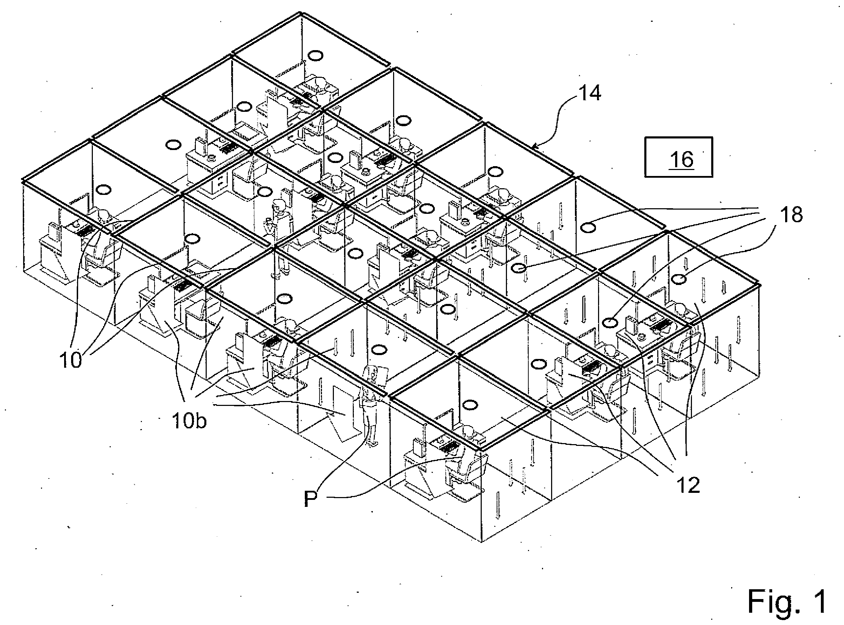

Figur 1 zeigt ein erstes Ausführungsbeispiel eines Systems gemäß der Erfindung, und zwar ein System zur Verhinderung oder Minimierung der Verbreitung von Viren in Raumluft in einem Großraumbüro. Das Großraumbüro hat einen in quadratische Raumsegmente aufgeteilten Grundriss mit in Reihen angeordneten Arbeitsplätzen und Gängen. Jeder Arbeitsplatz ist mit einem Arbeitstisch einem Stuhl und Ablagen ausgestattet. Die Erfindung ist aber auch auf andere Räume anwendbar, beispielsweise solche mit unterschiedlich großen Arbeitsplätzen oder Büros mit Open-Space-Konzept.

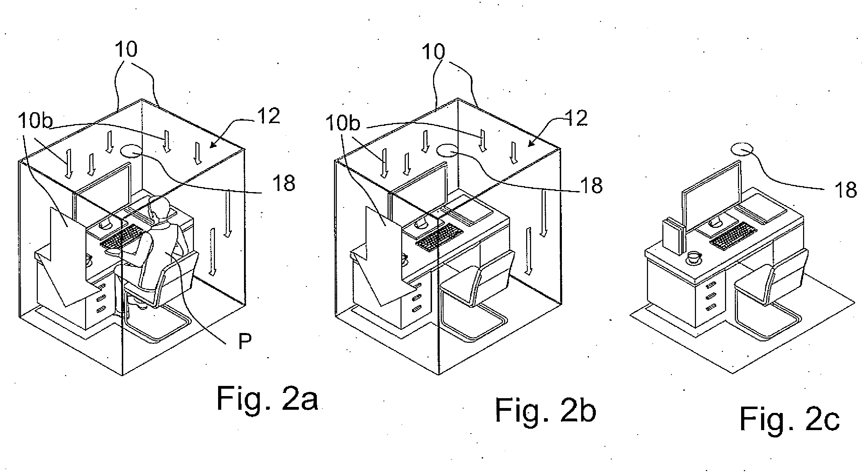

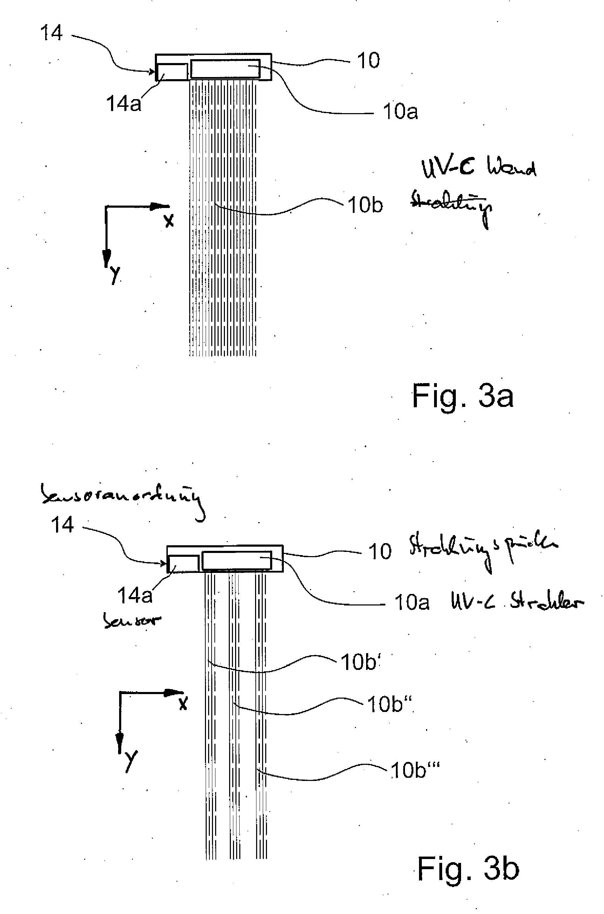

An der Decke des Raums ist eine gitterartige Anordnung von Strahlungsquellen 10 angebracht. Jede der Strahlungsquellen 10 ist eine Lichtleiste mit einem oder mehreren UV-C-Strahlern 10a (Fig. 3a, 3b), beispielsweise einer Quecksilberdampf-Entladungslampe, LEDs oder Laserdioden, und erzeugt jeweils ein wandartiges Strahlungsfeld 10b. Das Strahlungsfeld 10b kann insbesondere kurzwellige Far-UV-C-Strahlung mit Wellenlängen im Bereich von 207 - 222 nm enthalten. Durch geeignete Filter können schädliche Wellenlängen herausgefiltert werden. Zur Erzeugung der Far-UV-C-Strahlung kommen insbesondere Excimerlampen mit einer Kr-Cl-Gasmischung in Betracht. Im Folgenden werden die wandartigen Strahlungsfelder 10b der Einfachheit halber auch als UV-C-Wand bezeichnet. In Fig. 1, 2a und 2b sind die eigentlich unsichtbaren UV-C-Wände 10b als vertikal nach unten gerichtete, weiße Pfeile dargestellt.

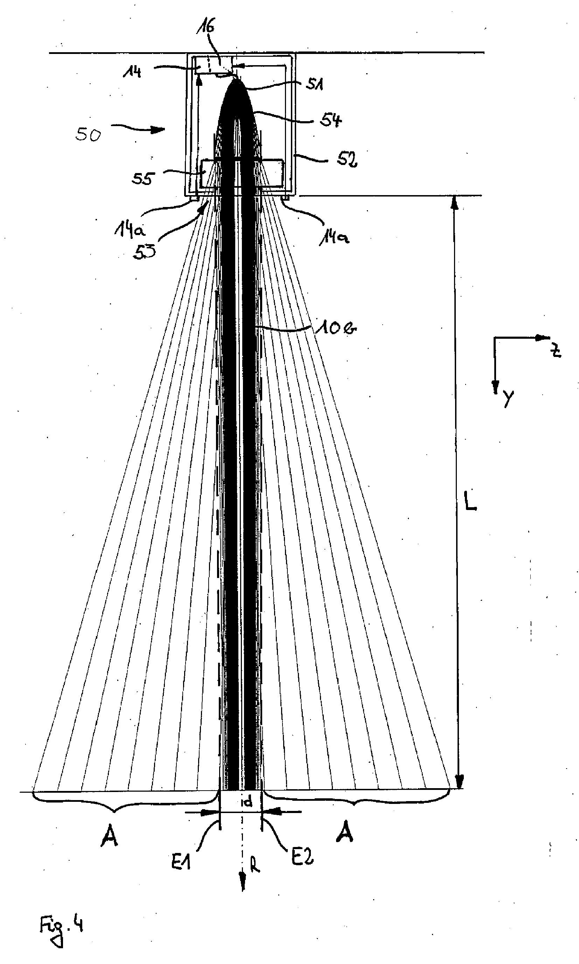

Zur Erzeugung eines wandartigen Strahlungsfelds 10b kann Strahlung optisch oder durch Spaltblenden zu parallelen Strahlen gebündelt bzw. kollimiert werden, wie dies unter Bezugnahme auf die Figuren 4 bis 7 nachfolgend noch detaillierter erläutert wird. Alternativ oder ergänzend dazu kann das Strahlungsfeld 10b durch eine Anordnung von parallelen Laserstrahlen mit lateral überlappenden Strahlungsprofilen erzeugt werden. Eine weitere Alternative wäre ein oder mehrere schnell in einer Fläche hin- und herbewegte bzw. gescannte Laserstrahlen - ähnlich wie bei Barcode-Scannern - wobei die Scangeschwindigkeit und der Strahldurchmesser so aufeinander abgestimmt sind, dass jedes durch die UV-C-Wand 10b hindurch diffundierende Aerosol einer hinreichenden Strahlungsdosis ausgesetzt ist.

Die Raumsegmente 12 sind jeweils durch UV-C-Wände 10b voneinander abgegrenzt. In dem in Fig. 1 dargestellten Ausführungsbeispiel ist jedes Raumsegment 12 von vier UV-C-Wänden 10b begrenzt.

Auch wenn in Fig. 1 solche Sonderfälle nicht dargestellt sind, bilden vorhandene solide Wände des Raums bilden Begrenzungen der Raumsegmente 12, so dass Raumsegmente 12 am Rand oder in Ecken des Raums neben den vorhandenen soliden Wänden nur durch drei bzw. zwei weitere UV-C-Wände 10b abgegrenzt werden müssen. Es sind auch Ausgestaltungen der Erfindung denkbar, in denen ein mit soliden Wänden abgegrenzter Raum, z.B. ein Einzelbüro, ein akustisch abgeschirmter Besprechungsbereich etc. ein Raumsegment 12 bilden, welches Aerosole mit anderen Raumsegmenten 12 nur durch eine Türöffnung oder einen Zugang austauschen kann. In diesem Fall ist es ausreichend, nur die betreffende Türöffnung bzw. den Zugang mit einer UV-C-Wand 10b von den restlichen Raumsegmenten 12 abzuschirmen.

Strukturen wie halbhohe Wände, Raumteiler oder dergleichen können durch eine UV-C-Wand 10b bis zur Decke fortgesetzt bzw. erweitert werden. In diesem Fall könnten die Strahlungsquellen 10 auch auf der Oberseite der betreffenden Struktur montiert werden und nach oben zur Decke hin strahlen.

In den Lichtleisten 10 sind zudem Sensoren 14a (Fig. 3a, 3b) einer Sensoranordnung 14 zum Detektieren einer Bewegung oder einer Anwesenheit einer oder mehrerer Personen P in dem Raum angeordnet.

Eine zentrale Steuerung 16 ist durch eine geeignete Software dazu ausgelegt, die eine oder mehreren Strahlungsquellen 10 abhängig von wenigstens der Anwesenheit der Person P ein- oder auszuschalten, wie dies im Folgen- den detaillierter beschrieben wird. Die Steuerung 16 kommuniziert dazu über Signalleitungen oder drahtlos, beispielsweise über WLAN, mit den Strahlungsquellen 10.

Die Steuerung 16 wertet die Positions- und Bewegungsdaten der Personen P aus und berechnet Wahrscheinlichkeiten für verschiedene Wege bzw. Bewegungen der Personen P. Wenn eine Person P ruhig und in hinreichender Entfernung von allen UV-C-Wänden 10b an ihrem Arbeitsplatz sitzt, ist es unwahrscheinlich, dass sie im nächsten Sekundenbruchteil eine der UV-C- Wände 10b durchquert. Wenn die Person aber zügig durch einen Gang läuft, der durch mehrere UV-C-Wände 10b in Raumsegmente 12 aufgeteilt ist, ist der Zeitpunkt des Durchquerens der nächsten UV-C-Wand 10b gut vorhersehbar. Wegen der gesundheitlichen Risiken werden die Strahlungs- quellen 10 bereits bei einer geringen Wahrscheinlichkeit abgeschaltet, wobei der Schwellenwert bei der Verwendung von Far-UV-C-Strahlen wegen der geringeren Risiken auf einen höheren Wert gesetzt werden kann als bei längerwelligen UV-Strahlungstypen.

Wenn anhand der von der Sensoranordnung 14 detektierten Bewegungsdaten eine hinreichende Wahrscheinlichkeit für das Durchqueren der UV-C- Wand 10b festgestellt wurde, schaltet die Steuerung 16 die betreffende Strahlungsquelle 10 oder zumindest Teile hiervon ab. Zum Abschalten lediglich eines Teils wird eine Gesamtheit von Leuchtmitteln, die als Strahler in der Strahlungsquelle 10 vorgesehen sind in Gruppen und gegebenenfalls Untergruppen unterteilt, wie dies nachfolgend in der detaillierteren Erläuterung einer Leuchte als Strahlungsquelle 10 ausgeführt wird. Wird dagegen ein sich längs in der Strahlungsquelle 10 erstreckendes Leuchtmittel verwendet, ist lediglich ein Abschalten des gesamten Leuchtmittels möglich. Alternativ können schaltbare blenden vorgesehen sein, mit denen bestimmte Bereiche abgeschattet werden.

Die Personen P können sich daher frei im Raum bewegen. Wenn die Person P dabei eine Grenzfläche zwischen zwei Raumsegmenten 12 durchquert, schaltet die Steuerung 16 die diese Grenzfläche bildende UV-C-Wand 10b ab und schaltet die UV-C-Wand 10b wieder ein, wenn sich die Person vollständig im zweiten Raumsegment 12 befindet.

Während sich eine oder mehrere Personen P sich in dem betreffenden Raumsegment 12 aufhält, bleiben in der Regel die betreffenden Strahlungs- quellen 10 aktiv, so dass Viren und Bakterien in Tröpfchen oder Aerosolen beim Verlassen des Raumsegments 12 abgetötet werden. Dadurch werden Personen P, die sich in unterschiedlichen Raumsegmenten 12 befinden, von einander abgeschirmt. Da die Strahlungsquellen 10 aktiv bleiben, während sich Personen P in den Raumsegmenten 12 aufhalten, können zur Vermeidung gesundheitlicher Schäden durch Streulicht Absorber-Leisten auf dem Boden angebracht sein, die das aus den Strahlungsquellen 10 einfallende UV-C-Licht absorbieren.

Nur wenn eine Person P das Raumsegment 12 durch eine UV-C-Wand 10b betreten oder verlassen möchte, wird die der entsprechenden UV-C-Wand 10b zugeordnete Strahlungsquelle 10 deaktiviert.

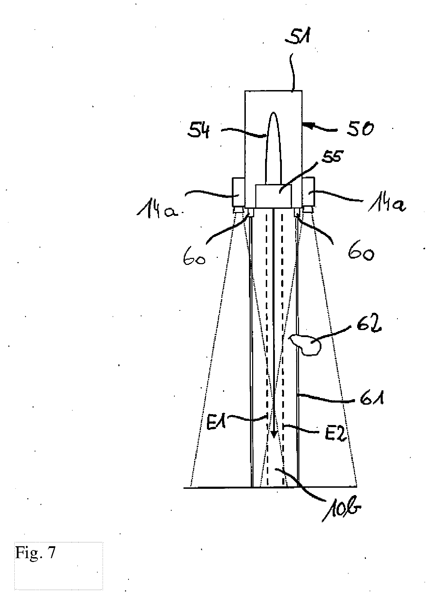

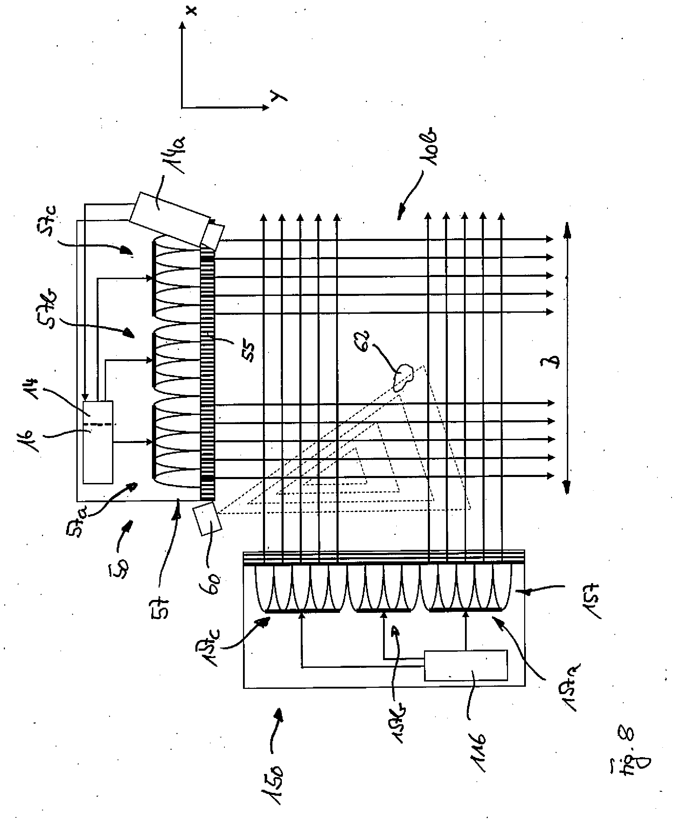

Alternativ oder als Ergänzung zu der vorstehend erläuterten Erfassung von Bewegungen einer Person im Raum ist bevorzugt vorgesehen, dass das Eindringen einer Person oder eines Gegenstands in eine direkt benachbart zu dem Strahlungsfeld vorgesehene Sicherheitszone durch die Sensoranordnung erkannt wird. Eine solche Vorgehensweise wird nachfolgend unter Bezugnahme auf die Figuren 7 und 8 noch erläutert. Ein Eindringen beliebiger Objekte in die Sicherheitszone wird dabei ausgewertet um nicht nur eine direkte Bestrahlung einer Person oder eines Körperteils einer Person zu verhindern, sondern auch eine mögliche Reflexion zu vermeiden, durch die ebenfalls anwesende Personen, selbst wenn sie Abstand zu dem Strahlungsfeld haben, geschädigt werden könnten. Das überwachen einer Sicherheitszone, die unmittelbar benachbart zu der UV Wand ausgebildet ist bringt insbesondere den Vorteil mit, dass eine sehr dicht an diese Sicherheitszone heranreichende Bewegung noch nicht zu einem Abschalten zumindest eines Teils der UV Wand führt. Ein denkbares Szenario ist die Anordnung der erfindungsgemäßen Strahlungsquelle 10 bzw. der nachfolgend noch erläuterten Leuchte, über einem Tisch in einem Restaurant. Typische Bewegungen, die von an diesem Tisch sitzenden Personen ausgeführt werden befinden sich in Bereichen, die hinreichend Abstand von der UV Wand haben. Greift dagegen eine Person über den Tisch, beispielsweise um seinem Gegenüber etwas anzureichern, so wird dies bei Eindringen in die Sicherheitszone erkannt und der entsprechende Teil oder die gesamte Strahlungsquelle 10 abgeschaltet.

Mittig an der Decke sind in den Raumsegmenten 12 weitere Strahlungsquellen 18 mit Viren deaktivierender bzw. desinfizierender Wirkung angebracht.

Die Steuerung 16 ist dazu ausgelegt, die weiteren Strahlungsquellen 18 für ein vorgegebenes Zeitintervall zu aktivieren, wenn sich keine Person in dem Raumsegment 12 befindet. Auch diese Strahlungsquellen 18 werden abgeschaltet, wenn eine Person P das betreffende Raumsegment 12 betritt. Um für die Person P kenntlich zu machen, ob die Desinfektion des betreffenden Raumsegments 12 abgeschlossen ist, kann eine Leuchtdiode oder ein Ampelsystem vorgesehen sein. Es sind weitere Ausgestaltungen der Erfindung denkbar, in welchen die Sensoranordnung 14 Sensoren umfasst, die in die Strahlungsquellen 18 integriert sind. Die Strahlungsquellen 18 können in Deckenverkleidungskacheln, Lampen oder Lüftungsgitter integriert sein oder mit anderen Geräten, z.B. Rauchmeldern, in ein Gehäuse integriert sind.

Die Fig. 2a - 2c zeigen ein einzelnes Raumsegment 12 des Systems aus Fig. 1 in drei verschiedenen Zuständen.

In dem in Fig. 2a dargestellten Arbeits-Zustand arbeitet eine Person P in dem von vier UV-C-Wänden 10b abgegrenzten Raumsegment 12. Alle vier UV-C-Wände 10b sind eingeschaltet, so dass in Aerosolen enthaltene Keime beim Durchqueren der Grenzflächen zwischen benachbarten Raumsegmenten 12 inaktiviert werden.

In dem in Fig. 2b dargestellten Desinfektions-Zustand hat eine Person P in dem Raumsegment 12 gearbeitet und es verlassen. Beim Verlassen des Raumsegments 12 aufgrund der detektierten Bewegung der Person P eine der vier UV-C-Wände 10b abgeschaltet (nicht dargestellt). Alle vier UV-C-Wände 10b sind eingeschaltet, so dass keine aktiven Keime austreten können. Zusätzlich wird die mittig an der Decke angebrachte Strahlungsquelle 18 für einen vorgegebenen Zeitraum aktiviert, um auch die auf den Oberflächen des Arbeitsplatzes und innerhalb des Raumsegments 12 schwebenden Keime abzutöten.

In dem in Fig. 2c dargestellten Ruhe-Zustand ist die Desinfektion abgeschlossen und keine Person P hält sich in dem Raumsegment 12 auf. Zum Energiesparen werden alle vier UV-C-Wände 10b und auch die mittig an der Decke angebrachte Strahlungsquelle 10 abgeschaltet.

Fig. 3a zeigt eine schematische Schnittansicht einer Strahlungsquelle 10 und eines wandartigen Strahlungsfelds 10b nach dem ersten Ausführungsbeispiel der Erfindung. Das Strahlungsfeld 10b hat eine im Rahmen der optischen Möglichkeiten konstante Dicke von etwa 1 cm.

Wie oben beschrieben implementiert die Steuerung 16 ein Verfahren zur Verhinderung oder Minimierung der Verbreitung von Viren in Raumluft mit einer oder mehreren Strahlungsquellen 10 in einem Raum. Das Verfahren umfasst das Detektieren einer Bewegung oder einer Anwesenheit einer oder mehrerer Personen P in dem Raum und das automatische Ein- oder Aus- schalten einer oder mehrerer Strahlungsquellen 10 abhängig von wenigstens der Anwesenheit der Person P.

Nach dem Verfahren wird die betreffende Strahlungsquelle 10 abgeschaltet, wenn die aus den von der Sensoranordnung 14 detektierten Bewegungsdaten es wahrscheinlich erscheinen lassen, dass eine der Personen P das betreffende Strahlungsfeld 10b durchqueren möchte oder aber eine Person bzw ein Objekt in die Sicherheitszone eingedrungen ist..

Die Figur 3b zeigt ein weiteres Ausführungsbeispiel der Erfindung. Um Wiederholungen zu vermeiden, beschränkt sich die nachfolgende Beschreibung dieser weiteren Ausführungsbeispiele im Wesentlichen auf Unterschiede zu dem ersten Ausführungsbeispiel der Erfindung. Wegen der unveränderten Merkmale wird der Fachmann auf die Beschreibung zum ersten Ausführungsbeispiel verwiesen. Für gleich oder ähnlich wirkende Merkmale der weiteren Ausführungsbeispiele werden gleiche Bezugszeichen verwendet, um die Ähnlichkeiten zu betonen.

In dem in Figur 3b dargestellten Ausführungsbeispiel sind die Strahlungs- quellen 10 jeweils zur Erzeugung mehrerer, parallel verlaufender Strahlungsfelder 10b' - 10b''' ausgelegt, die beispielsweise eine Dicke von weniger als 50mm, bevorzugt weniger als 40mm, weiter bevorzugt 25mm oder 1 mm und einen Abstand z.B. von 1 mm haben können. Größere Abstände zwishen den parallelen Strahlungsfeldern 10b' - 10b''' sind möglich, erhöhen jedoch den Platzbedarf. Auch eine andere Anzahl von Strahlungsfeldern 10b' - 10b''' ist denkbar.

Zur Ausbildung des vorstehend beschriebenen Systems werden als Strahlungsquelle 10 bevorzugt Leuchten 50 eingesetzt, die der in Figur 4 dargestellten Ausführungsformen entsprechen. Es ist zu beachten, dass die Darstellungen lediglich schematisch sind und keinerlei Anspruch auf eine korrekte Wiedergabe von Größenverhältnissen erheben. Wo es angebracht scheint werden vielmehr die Größenverhältnisse so angepasst, dass die Erfindung leicht nachvollziehbar ist.

Die in der Figur 4 dargestellte Leuchte 50 weist eine Mehrzahl von Leuchtmitteln 51 auf, wobei durch die Schnittdarstellung lediglich eines der Leuchtmittel 51 in der Figur 4 zu erkennen ist. Die Leuchte 50 weist zudem ein Gehäuse 52 auf, welches für UV-C Strahlung undurchlässig ist. Das Gehäuse 52 verfügt über eine Austrittsöffnung 53, durch die von dem Leuchtmittel 51 erzeugte die UV-C Strahlung aus dem Leuchtengehäuse 52 austreten kann. Im dargestellten Ausführungsbeispiel ist die Leuchte 50 zur Montage an einer Decke eines Raums vorgesehen. Selbstverständlich kann auch eine Montage an einer Wand eines Raums erfolgen. Die nachfolgend beschriebene Funktion ist unabhängig von der Orientierung der Leuchte 50.

Das Leuchtmittel 51 emittiert keimtötende UV-C Strahlung, welche durch einen Reflektor 54 kollimiert wird. Der Reflektor 54 ist ein Beispiel für ein optisches Element, mit dem von dem Leuchtmittel 51 emittierte Strahlung kollimiert werden kann. Andere optische Elemente, wie beispielsweise entsprechend ausgebildete Linsen, sind ebenfalls denkbar. Die Auswahl und Ausprägung des optischen Elements, das zum Kollimieren der emittierten Strahlung eingesetzt wird kann zum Beispiel nach wirtschaftlichen oder fertigungstechnischen Aspekten oder dem Wirkungsgrad erfolgen.

Die an der Innenseite des rotationssymmetrischen Reflektors 54 reflektierte Strahlung wird als kollimierte Strahlung bezeichnet. Dieser kollimierte Anteil der von dem Leuchtmittel 51 emittierten Strahlung tritt aus der Austrittsöffnung 53 aus, wobei durch das Kollimieren die kollimierte Strahlung innerhalb eines gedachten Zylinders mit dem Durchmesser d in Richtung der Y-Achse aus der Austrittsöffnung 53 austritt. Die Geometrie des Reflektor 54 ist so gewählt, dass für typische Raumhöhen oder Raumdimensionen, die mit einer maximalen Länge L gleich 5 m abgeschätzt werden können, der Durchmesser d der kollimierten Strahlung stets kleiner als 8 cm, bevorzugt 5 cm ist. Es ist zu beachten, dass diese Angaben lediglich bevorzugte Werte darstellen.

Die Längsachse der Leuchte 50 steht senkrecht auf Zeichenebene. Die im Schnitt dargestellte Anordnung des Leuchtmittels 51 sowie des Reflektor 54 wiederholt sich entlang der Längsachse der Leuchte 50, wobei die in der Leuchte 50 angeordnete Mehrzahl von Leuchtmittel 51 und jeweils zugeordneten Reflektoren 54 entlang einer geraden Linie angeordnet sind. Damit bilden die in der Leuchte 50 angeordneten Leuchtmittel 51 und die jeweils zugeordneten Reflektoren 54 bei der dargestellten Ausführungsform der Figur 4 gemeinsam eine einzige Gruppe aus, wobei die Abstrahlrichtungen R aller einzelnen Leuchtmittel 51 und ihrer zugeordneten Reflektoren 54 parallel zueinander ausgebildet sind und in einer Ebene liegen.

Wie es nachfolgend noch genauer erläutert wird, sind die benachbarten Reflektoren 54 so entlang dieser Linie angeordnet, dass die durch die benachbarten Reflektoren 54 jeweils kollimierte Strahlung überlappt und somit die Gesamtheit der kollimierten Strahlung der Leuchtmittel 51 das wandartiges Strahlungsfeld 10b als Barriere für Viren erzeugt. Die maximale Ausdehnung dieses wandartigen Strahlungsfelds 10b in einer Richtung senkrecht zur Längserstreckung der Leuchte 50 sowie zur Abstrahlrichtung, also die Ausdehnung in Richtung der z-Achse, wird von zwei gedachten Ebenen E1 und E2 begrenzt. Der Abstand dieser beiden Ebenen E1 und E2 entspricht damit dem Durchmesser d des gedachten Zylinders.

Die Leuchtmittel 51 und die Reflektoren 54 sind dabei so aufeinander abgestimmt, dass die Intensität der kollimierten Strahlung zum Abtöten von Keimen ausreicht. Außerhalb der so gebildeten UV Wand 10b ist dagegen Strahlung nur mit unkritischer Intensität vorhanden. Diese Strahlung entsteht durch den nicht kollimierten Anteil der vom Leuchtmittel 51 emittierten Strahlung, also dem ohne Reflexion aus dem Reflektor 54 austretenden Anteil. In der Figur 4 ist dieser Strahlungsanteil außerhalb des Bereichs zwischen den Ebenen E1 und E2 durch einzelne Strahlen gezeigt.

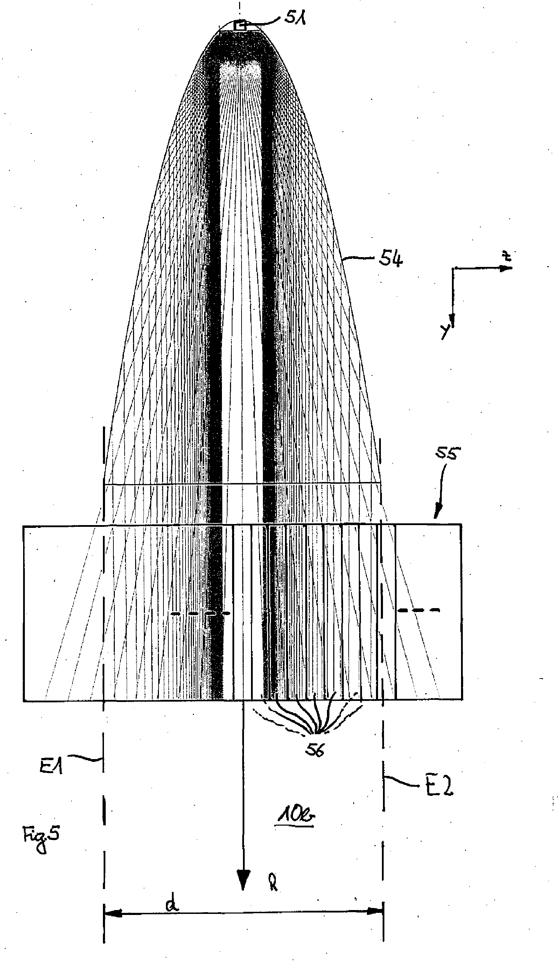

Zur Verbesserung der Sicherheit wird vorzugsweise eine Abblendeinrichtung 55 im Bereich der Austrittsöffnung 53 der Leuchte 50 angeordnet. Die Abblendeinrichtung 55 kann dabei selbst die Austrittsöffnung 53 bilden oder aber innerhalb, aber auch außerhalb des Gehäuses 52 der Leuchte 50 angeordnet sein. Die Wirkungsweise der Abblendeinrichtung 55 wird nachfolgend unter Bezugnahme auf die Figur 5 genauer erläutert. Durch die Abblendeinrichtung 55 wird sichergestellt, dass der nicht kollimierte Anteil der vom Leuchtmittel 51 emittierten Strahlung abgeschattet wird, also am Austritt aus der Öffnung 53 gehindert wird. Wie es in der Figur 4 dargestellt ist, würde dieser direkt vom Leuchtmittel 51 abgestrahlte Anteil außerhalb der durch die Ebenen E1 und E2 begrenzten UV Wand die Bereiche A beleuchten. In diesen Bereichen könnte sich also, sofern eine kritische Intensität der dort vorhandenen UV-C Strahlung auftritt, keine Personen ohne Sicherheitsrisiko aufhalten. Unabhängig von der genauen Positionierung der Abblendeinrichtung 55 ist die Abblendeinrichtung 55 so dimensioniert und positioniert, dass sämtliche, das Gehäuse 52 der Leuchte 50 verlassende Strahlung die Kanäle der Abblendeinrichtung 55 passieren muss.

In der Figur 4 ist ferner dargestellt, dass an der Leuchte 50 Sensoren 14a angeordnet sind, die Teil einer Sensoranordnung sind, deren Informationsverarbeitung in die Steuerung 16 integriert sein kann. Im dargestellten Ausführungsbeispiel ist die Steuerung 16 in die Leuchte 50 integriert Zumindest aber werden Signale der Sensoren 14a oder aber bereits ein Ergebnis einer Auswertung an die Steuerung 16 übermittelt, sodass durch diese auf Basis der ausgewerteten Signale ein An- bzw. Abschalten der Leuchtmittel 51 möglich ist.

Die Figur 5 zeigt in einer vergrößerten Darstellung das Leuchtmittel 51 und den Reflektor 54 zusammen mit der Abblendeinrichtung 55. Schematisch sind Kanäle 56 der Abblendeinrichtung 55 gezeigt, die parallel zur Abstrahlrichtung R verlaufen und so die kollimierte Strahlung passieren lassen, während Strahlungsanteile, die schräg zur Abstrahlrichtung R verlaufen, an Innenwänden der Kanäle 56 einfallen. Um sicherzustellen, dass selbst von einem möglicherweise an einer Innenwand reflektierten Strahl keine Gefahr ausgeht, sind die Innenwände der Kanäle 56 mit einem UV-C Strahlung absorbierenden Material beschichtet oder aber die Abblendeinrichtung 55 ist aus einem solchen Material gefertigt.

Die Abblendeinrichtung 55 kann entweder für jeden Reflektor 54 individuell vorgesehen sein, und beispielsweise die Öffnung des Reflektors 54 bedecken, oder als gemeinsame Abblendeinrichtung für die Gesamtheit der Reflektoren 54.

Ferner ist zu beachten, dass für die detaillierte Erläuterung der Leuchte 50 davon ausgegangen wird, dass eine Mehrzahl von individuellen Leuchtmitteln 51 gemeinsam die schließlich die UV wandbildende Strahlung imitieren. Es kann jedoch auch ein sich in Längsrichtung erschreckendes Leuchtmittel zur Erzeugung der Strahlung eingesetzt werden.

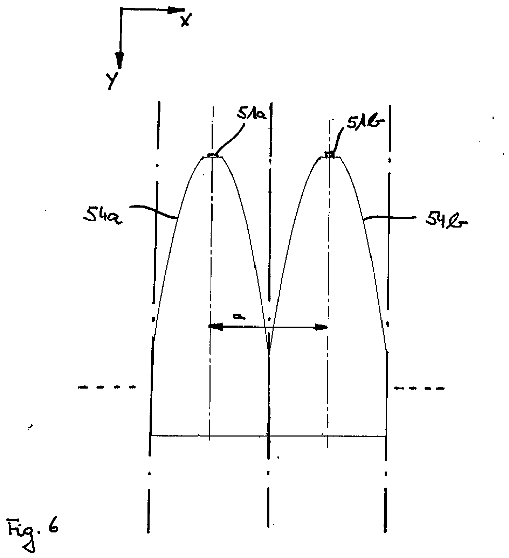

Die Figur 6 zeigt stark vereinfacht einen Schnitt durch die reflektierende Fläche benachbarter Reflektoren 54 in Form eines resten Reflektors 54a und eines zweiten Reflektors 54b. Die beiden Reflektoren 54a und 54b sind mit einem Abstand a in der Leuchte 50 angeordnet, der kleiner ist als der Durchmesser d des gedachten Zylinders bzw. des Abstands d der gedachten Ebenen E1 und E2 als Grenzen des wandartigen Strahlungsfelds 10b.

In dem dargestellten Ausführungsbeispiel wird davon ausgegangen, dass sämtliche Reflektoren 54, die in einer Leuchte 50 vorgesehen sind, identische Geometrien aufweisen. Die damit jeweils von einem Leuchtmittel 51 mit Hilfe seines zugeordneten Reflektors 54 emittierte, kollimierte Strahlung ist somit hinsichtlich ihrer Strahlungsgeometrie gleich. Grundsätzlich ist es auch denkbar unterschiedliche Geometrien für benachbarter Reflektoren 54 einzusetzen. Der Abstand der jeweiligen Symmetrieachsen bei Verwendung rotationssymmetrischer Reflektoren ist dann jeweils so anzupassen, dass die gedachten Zylinder, die die kollimierte Strahlung einhüllen, sich schneiden.

Durch das Sich-Schneiden der gedachten, die jeweils von einem optischen Element kollimierte Strahlung einhüllenden Zylinder stellt sicher, dass eine lückenlose Barriere aufgebaut wird.

Wie es weiter oben bereits angedeutet wurde, ist es für den Betrieb der erfindungsgemäßen Leuchte 50 bzw. des gesamten Systems erforderlich, das zuverlässig verhindert werden kann, dass die emittierte UV-Strahlung auf Personen trifft, hierdurch zu Schaden kommen könnten. Neben der bereits im Zusammenhang mit dem System erläuterten Vorhersage von Bewegungen von Personen oder Fassungen des Orts, an dem sich Personen aufhalten kann auch das unmittelbare Eindringen in eine Sicherheitszone, die angrenzend an das Strahlungsfeld also benachbart zu den Ebenen E1 und E2 definiert ist, erfasst werden. Figur 7 zeigt stark vereinfacht eine Sensoranordnung, mit der die Erfassung des Eindringens in eine solche Sicherheitszone möglich ist.

Mit Hilfe von Sensoren 14a werden in dem dargestellten Ausführungsbeispiel Reflexionen erfasst, die beim Auftreffen einer von einem sogenannten Strichlaser (Linienlaser) 60 emittierten Strahlung entstehen. Im dargestellten Ausführungsbeispiel wird davon ausgegangen, dass sich beidseits der UV-Wand 10b Personen aufhalten können wie dies typisch in einem Restaurant ist. Es ist daher zu beiden Seiten der UV-Wand 10b ein Strichlaser 60 sowie eine zugeordnete Kamera als Sensor 14a zu Erfassung der Reflexionen des Laserstrahls vorgesehen. Links der UV Wand 10b ist zu erkennen, dass die emittierte Laserstrahlung des links angeordneten Strichlasers 60 zum Beispiel auf den Fußboden oder andere im wesentlichen unveränderliche Einrichtungsgegenstände fällt. Diese Reflexion wird durch den Sensor 14a erfasst.

Auf der rechten Seite der UV Wand 10b ist dagegen gezeigt, dass ein Objekt 62, dies kann beispielsweise ein Finger einer Person sein oder ein von der Person bewegter Gegenstand, sich der UV Wand 10b nähert und damit in eine Bereich kommt, indem er einen Teil des vom Strichlaser 60 ausgestrahlten Laserlichts reflektiert. Bis zum Eindringen in die Ebene des von dem Strichlaser 60 emittierten Laserlichts wurde auch hier das Licht lediglich vom Boden reflektiert. Unmittelbar bei Eindringen des Objekts 62 wird dagegen die Reflexion verändert, was durch den Sensor 14a erfasst wird. Aus der Änderung kann auf das Eindringen eines Objekts in die Sicherheitszone geschlossen werden. Die Sicherheitszone ist dabei der Raum von der UV Wand 10b bzw. der begrenzenden Ebene E2 bis einschließlich der von dem auf der Seite der Ebene E2 angeordneten Strichlaser 60 parallel zur Ebene E2 ausgesandten.

Auf Seite der anderen Ebene E1 ist ebenso eine Sicherheitszone ausgebildet. Die Ausbildung einer 2. Sicherheitszone kann dann entfallen, wenn die Leuchte nahe einer Wand und parallel zu dieser angebracht ist, sodass ein Eindringen in den Bereich der UV Wand 10b von dieser Seite aus unmöglich ist.

Werden durch die Leuchte 50 mehrere parallel angeordnete Strahlungsfelder 10b' - 10b''' erzeugt, so sind die Sicherheitszonen lediglich benachbart zu den jeweils äußersten Strahlungsfeld an vorzusehen. Die durch die mehreren Strahlungsfelder 10b' - 10b''' größer werdenden stirnseitigen Abstände sind dann mit separaten Schutzmaßnahmen abzusichern. Diese können den oben beschriebenen, parallel zu den Strahlungsfeldern angeordneten Sicherheitseinrichtungen entsprechen. Erstreckt sich die Ausdehnung der Leuchte zwischen 2 Wänden oder anderen, UV Licht Abstimmenden baulichen Objekten, so kann auch dann auf eine Absicherung der Stirnseiten verzichtet werden.

Die Funktion dieser Sicherheitseinrichtungen wird nun anhand Darstellung in Figur 8 erläutert. Die in der Figur 8 dargestellte Anordnung zeigt die Leuchte 50, wie sie unter Bezugnahme auf die Figur 7 bereits erläutert wurden, zusammen mit dem Sensor 14a sowie dem Strichlaser 60. Das von dem Strichlaser 60 ausgesandte Laserlicht wird durch die gestrichelten Dreiecke schematisch dargestellt. Die Ebene, in der das Laserlicht ausgesandt wird ist dabei parallel und beanstandet zu der kollimierten Strahlung, die durch die Gesamtheit von Leuchtmitteln 51 der Leuchte 50 ausgesandt werden kann. Der reflektierte Anteil des ausgestrahlten Laserlichts des Strichlasers 60 wird durch den Sensor 14a erfasst und einer Auswertung zugeführt. Wie vorstehend bereits erläutert, wird bei der Auswertung insbesondere die Änderung der Reflexion des Laserlichts erfasst, sodass ein Eindringen eines Objekts in den durch den Strichlaser 60 beleuchteten Bereich durch die Sensoranordnung bzw. deren Informationsverarbeitungsvorrichtung 14 erkannt werden kann. Die Sensoranordnung 14 kann insbesondere einen Prozessor aufweisen, oder andere Einrichtungen zur Verarbeitung der von dem Sensor 14a übermittelten Informationen. Diese Vorrichtung zur Datenverarbeitung kann mit der Steuerung 16 gemeinsam realisiert sein. Im dargestellten Ausführungsbeispiel ist die Steuerung 16 einschließlich des Informationsverarbeitungsteils der Sensoranordnung 14 in der Leuchte 50 integriert.

In der beispielhaft dargestellten Leuchte 50 sind insgesamt 14 Leuchtmittel 51 entlang einer geraden Linie angeordnet, wobei jedem dieser Leuchtmittel 51, die in der Figur 8 der besseren Übersichtlichkeit wegen nicht separat dargestellt sind, ein optisches Element in Form eines Reflektor 54 (hier ohne Bezugszeichen dargestellt) zugeordnet. Das emittierte UV-Licht Licht wird stellvertretend durch die als Pfeile dargestellten Abstrahlrichtungen angegeben. Die Abstrahlrichtungen der Leuchtmittel und ihrer zugeordneten optischen Elemente sind, wie aus der Zeichnung unmittelbar ersichtlich, parallel zueinander ausgerichtet. Ferner liegen sämtliche Abstrahlrichtungen der Leuchtmittel der Leuchte 50 in einer Ebene. Somit bilden alle Leuchtmittel der Leuchte 50 gemeinsam eine Gruppe von Leuchtmitteln aus.

Alternativ zu einer Leuchte 50, wie sie dargestellt ist, die lediglich eine einzige Gruppe von Leuchtmitteln aufweist, können auch mehrere Gruppen von Leuchtmitteln vorgesehen sein. Innerhalb einer Gruppe sind die Leuchtmittel und ihre zugeordneten Reflektoren dann ebenfalls wieder so angeordnet, dass ihre Abstrahlrichtungen parallel zueinander sind und in einer Ebene liegen. Die Ebenen verschiedener Gruppen können dabei parallel und beanstandet zueinander angeordnet sein oder aber einen Winkel aufweisen.

Für die Gruppe von Leuchtmitteln und ihre zugeordneten optischen Elemente der Leuchte 50 ist gezeigt, dass die Gruppe in drei Untergruppen 57a, 57b und 57c unterteilt ist. Jede dieser Untergruppen 57a, 57b und 57c enthält eine Mehrzahl von Leuchtmitteln sowie deren zugeordnete optische Elemente. Die Untergruppen 57a, 57b und 57c lassen sich einzeln durch die Steuerung 60 ansteuern, also an- und abschalten.

Wird nun ein Objekt 62 beim Eindringen in die durch den Strichlaser 60 beleuchtete Ebene aufgrund der durch den Sensor 14a erfassten Signale erkannt, so wird aus den von dem Sensor 14 A an die Steuerung 16 bzw. die dort integrierte Informationsverarbeitungsvorrichtung der Sensoranordnung 14 übermittelten Signale die Position des Objekts 62 ermittelt.

Es ist zu beachten, dass in der Figur 8 lediglich ein Strichlaser 60 sowie ein Sensor 14a gezeigt sind, wobei es aber besonders bevorzugt ist, eine Mehrzahl solcher Kombinationen von Strichlasern und Sensoren 14a vorzusehen, deren Erfassungsrichtungen einen Winkel von ungleich 0° bzw. 180° aufweisen. Mit Hilfe einer solchen Kombination von Anordnungen ist die Positionsbestimmung des Objekts 62 in zwei Dimensionen möglich. Ferner ist es bei Verwendung zweier solcher Anordnungen auch möglich, ein weiteres Objekt, das möglicherweise im Schatten des gezeigten Objekts 62 liegt, separat zu detektieren.

Bei Verwendung lediglich eine Anordnung kann dagegen die Position des Objekts 62 zumindest in einer Richtung (x-Achse) bestimmt werden. Die erkannte Position wird in der Steuerung 16 ausgewertet und diejenige Untergruppe 57a, 57b oder 57c abgeschaltet, deren emittierte, kollimierte Strahlung das Objekt 62 treffen würde. Im dargestellten Ausführungsbeispiel ist dies die mittlere Gruppe 57b. Es ist zu beachten, dass unter dem Begriff "Position" nicht nur ein Mittelpunkt eines erfassten Objekts 62, sondern auch dessen Ausdehnung verstanden wird. Liegt also ein erkanntes Objekt 62 nicht vollständig im Bereich des von einer Untergruppe 57a, 57b oder 57c abgestrahlten Lichts, so wird aufgrund der Positionserfassung einschließlich der Ausdehnung des Objekts 62 nicht nur eine Untergruppe abgeschaltet.

Sind dagegen die Positionskoordinaten für zwei Richtungen (x-Achse, y-Achse) bekannt, so kann eine zwiete Leuchte 150 eingesetzt werden, deren Aufbau im Grunde mit dem der Leuchte 50 vergleichbar ist, und deren Abstrahlrichtungen mit den Abstrahlrichtungen der Leuchte 50 einen Winkel ungleich 0° bzw. 180° einschließen. Bevorzugt stehen die Abstrahlrichtungen der Leuchten 50 und 150 senkrecht aufeinander. Die Abstrahlrichtungen bei der Leuchten liegen dabei in derselben Ebene, sodass die Sensoranordnung 14 einschließlich der Strichlaser 60 sowie der Sensoren 14a gemeinsam verwendet werden kann. Wird mit Hilfe der Sensoranordnung 14 die Position des Objekts 62 zweidimensional bestimmt, so kann nicht nur diejenige Untergruppe 57b der Leuchte 50 abgeschaltet werden, die im Bereich des erkannten Objekts 62 UV-Licht emittiert, sondern auch die entsprechende Untergruppe 157b der zweiten Leuchte 150. Wie aus der Zeichnung unmittelbar ersichtlich ist, wird damit lediglich ein relativ kleiner Bereich nicht mit UV-C Strahlung beleuchtet, sodass größere Lücken in der Barriere verhindert werden können.

Bei dem lediglich schematisch dargestellten Beispiel der Figur 8 weisen die Leuchten 50 und 150 eigene Steuerungen 16 bzw. 116 auf. Im Falle, dass eine gemeinsame Sensoranordnung 14 verwendet werden soll, um beide Leuchten 50 und 150 anzusteuern, ist eine Kommunikation zwischen der Steuerung 16 bzw. der Sensoranordnung 14 der Leuchte 50 und der Steuerung 116 der Leuchte 150 vorgesehen. Alternativ kann auch, wie es bereits bei der Figur 1 gezeigt wurde, eine externe Steuerung zum Ansteuern der Leuchtmittel in einer Mehrzahl von Leuchten 50,150, ... vorgesehen sein.

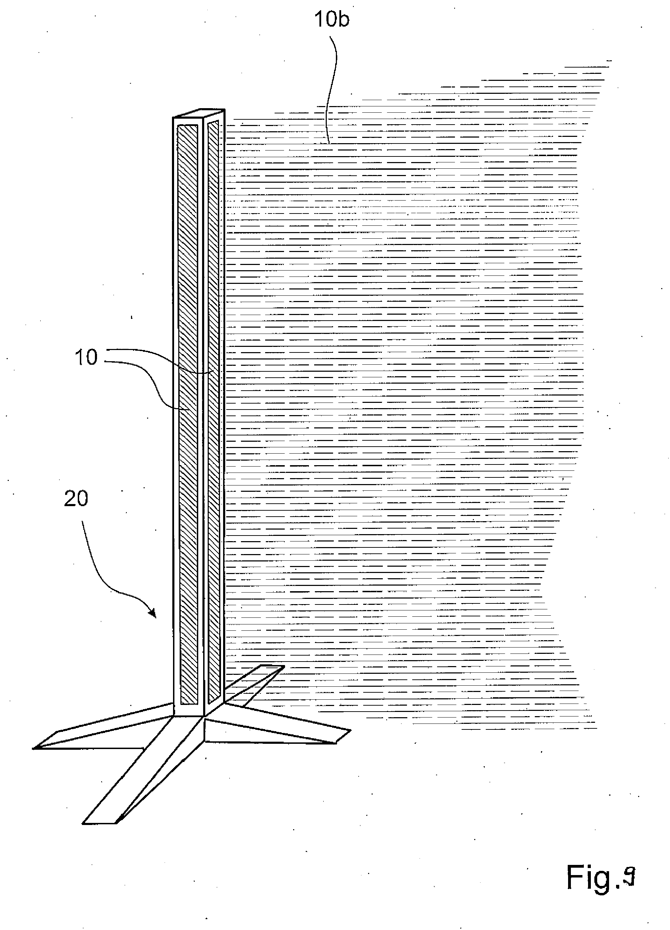

Fig. 9 zeigt eine weitere Ausgestaltung der Erfindung mit einem Ständer 20 für die Strahlungsquellen 10 eines erfindungsgemäßen Systems, die UV-C- Strahlung in einer horizontalen Richtung abstrahlen um so UV-C-Wände 10b zu bilden. Je nach Anwendungsbereich kann ein Ständer 20 mit einer, zwei, drei oder vier Strahlungsquellen 10 ausgestattet sind, die ausgehend von dem Ständer 20 bis zu vier in verschiedene Raumrichtungen ausstrahlende UV-C-Wände 10b erzeugen können. Das abgestrahlte UV-C-Licht kann von benachbarten Ständern oder zu diesem Zweck aufgestellten Absorber-Wänden oder Absorber-Ständern absorbiert werden.

In weiteren, nicht dargestellten Ausführungsformen der Erfindung können die Ständer vertikal nach unten strahlende Lichtleisten bzw. Strahlungsquellen halten. Ferner ist es denkbar, dass die Lichtleisten bzw. Strahlungsquellen auf dem Boden liegen und zur Decke hin strahlen.

## Patentansprüche

1. Leuchte zum Ausbilden einer Barriere für Krankheitserreger in Raumluft mit wenigstens einem UV-C Strahlung emittierenden Leuchtmittel (51), wobei die Leuchte (10, 50, 150) eine optische Einrichtung (54, 45a, 54b, 53) zum Kollimieren der von dem wenigstens einen Leuchtmittel (51, 51a, 51b) emittierten Strahlung zur Erzeugung eines Strahlungsfelds (10b) aufweist, dessen Dicke d um wenigstens eine Größenordnung kleiner als seine Länge L und Breite B ist.

2. Leuchte nach Anspruch 1,
**dadurch gekennzeichnet, dass**
die optische Einrichtung (54, 55) eine Abblendeinrichtung (55) zum Abblenden divergenter Strahlungsanteile umfasst.

3. Leuchte nach Anspruch 1 oder 2,
**dadurch gekennzeichnet, dass**
die Leuchte (50, 150) eine Mehrzahl von UV-C Strahlung emittierenden Leuchtmitteln (51, 51a, 51b) und die optische Einrichtung eine Mehrzahl von optischen Elementen (54, 54a, 54b) zum Kollimieren von Strahlung aufweist, die jeweils einem Leuchtmittel (51, 51a, 51b) zugeordnet sind, wobei die Mehrzahl von Leuchtmitteln (51, 51a, 51b) und diesen zugeordnete optische Elemente (54, 54a, 54b) wenigstens eine Gruppe bilden und die Abstrahlrichtungen R der von den Leuchtmitteln (51, 51a, 51b) emittierten kollimierten Strahlung innerhalb einer Gruppe parallel zueinander sind und in einer gemeinsamen Ebene liegen.

4. Leuchte nach Anspruch 3,
**dadurch gekennzeichnet, dass**
die optischen Elemente (54, 54a, 54b) so ausgebildet und in der Leuchte (50, 150) angeordnet sind, dass die jeweils kollimierte Strahlung benachbarter Leuchtmittel (51, 51a, 51b) überlappt.

5. Leuchte nach Anspruch 3 oder 4,
**dadurch gekennzeichnet,**
**dass** die Leuchtmittel (51, 51a, 51b) wenigstens einer Gruppe in Untergruppen (57a, 57b, 57c; 257a, 157b, 157c) unterteilt sind und die Leuchtmittel (51, 51a, 51b) dieser Untergruppen (57a, 57b, 57c; 257a, 157b, 157c) gemeinsam aber unabhängig von den Leuchtmitteln (51, 51a, 51b) anderer Untergruppen (57a, 57b, 57c; 257a, 157b, 157c) an- und abschaltbar sind.

6. Leuchte nach einem der Ansprüche 1 bis 5
**dadurch gekennzeichnet,**
**dass** die Leuchte (10, 50, 150) als Lichtleiste zur Decken- oder Wandmontage ausgestaltet ist.

7. Leuchte nach einem der Ansprüche 1 bis 6,
**dadurch gekennzeichnet, dass**
die Krankheitserreger deaktivierende UV-C-Strahlung, die zu einer UV-C-Wand gebündelt wird, eine Far-UV-C Strahlung mit einer Wellenlänge im Bereich von 200 - 222 nm, insbesondere 207 - 222 nm ist.

8. Leuchte nach einem der Ansprüche 1 bis 6,
**dadurch gekennzeichnet, dass**
die Krankheitserreger deaktivierende UV-C-Strahlung, die zu einer UV-C-Wand gebündelt wird, eine UV-C Strahlung mit einer Wellenlänge im Bereich von 223 - 280 nm ist.

9. System zur Verhinderung oder Minimierung der Verbreitung von Krankheitserregern in Raumluft mit einer oder mehrerer Strahlungsquellen (10) in Form einer oder mehrerer Leuchten (10, 50, 150) nach einem der Ansprüche 1 bis 3,
**dadurch gekennzeichnet, dass**
das System eine Sensoranordnung (14) zum Detektiereneines Eindringens einer oder mehrerer Personen (P) oder Gegenstände in eine benachbart zu dem Strahlungsfeld ausgebildete Sicherheitszone und eine Steuerung (16), die dazu ausgelegt ist, die eine oder mehreren Strahlungsquellen (10, 50, 150) abhängig von wenigstens der Anwesenheit der Person(en) (P) und/oder Gegenstände zumindest teilweise ein- oder auszuschalten, wobei die Steuerung (16) dazu ausgelegt ist, die betreffende Strahlungsquelle (10, 50, 150) zumindest teilweise abzuschalten, wenn die Sensoranordnung (14) ein Eindringen detektiert.

10. System nach Anspruch 9, **gekennzeichnet durch** freibewegliche Ständer (20) zum Halten einer oder mehrerer Strahlungsquellen (10, 50, 150).

11. System nach einem der Ansprüche 8 oder 9,
**dadurch gekennzeichnet, dass**
die eine oder die mehreren Strahlungsquellen (10, 50, 150) zur Anordnung entlang von Begrenzungen von Raumsegmenten (12) ausgelegt sind, wobei die Steuerung (16) dazu ausgelegt ist, die betreffenden Strahlungsquellen (10, 50, 150) zu aktivieren, wenn eine oder mehrere Personen (P) sich in dem betreffenden Raumsegment (12) aufhält und wenigstens eine der Strahlungsquellen (10, 250, 150) zu deaktivieren, wenn eine Person (P) das Raumsegment (12) betritt oder verlässt.

12. System nach Anspruch 10,
**dadurch gekennzeichnet, dass**
die Raumsegmente (12) ein regelmäßiges Raster bilden.

13. System nach einem der Ansprüche 10 oder 11,
**dadurch gekennzeichnet, dass**
innerhalb der Raumsegmente (12) weitere Strahlungsquellen (18) mit Krankheitserreger deaktivierender und/oder desinfizierender Wirkung angeordnet sind und die Steuerung (16) dazu ausgelegt ist, die weiteren Strahlungsquellen (10) zu aktivieren, wenn sich keine Person (P) in dem Raumsegment (12) befindet.

14. System nach einem der Ansprüche 8 bis 12,
**dadurch gekennzeichnet, dass**
die Sensoranordnung eine 3D-Kamera bzw. TOF-Kamera und/oder eine oder mehrere CCD-Kameras umfasst.

15. System nach einem der Ansprüche 8 bis 13,
**dadurch gekennzeichnet, dass**
die Sensoranordnung wenigstens eine Lichtquelle umfasst und eingerichtet ist, Änderungen im reflektierten Anteil des von der Lichtquelle imitierten und Objekten der Umgebung reflektierten Lichts zu erfassen.

16. System nach einem der Ansprüche 8 bis 14,
**dadurch gekennzeichnet, dass**
die Sensoranordnung eingerichtet ist ein Eindringen in die Sicherheitszone ortsaufgelöst zu ermitteln und die Steuerung (16) eingerichtet ist auf Basis des Orts des Eindringens ein Abschalten wenigstens eines Leuchtmittels (51, 51, 51b) vorzunehmen.

17. Verfahren zur Verhinderung oder Minimierung der Verbreitung von Viren in Raumluft mit einer oder mehreren Strahlungsquellen (10) in einem Raum,
**dadurch gekennzeichnet, dass**
das Verfahren das Erzeugen wenigstens eines Strahlungsfelds (10b), dessen Dicke d um wenigstens eine Größenordnung kleiner als seine Länge L und Breite B ist, durch Kollimieren der von einer odermehreren Leuchtmitteln emittierten Strahlung umfasst, wobei das Strahlungsfeld (10b) als Barriere für Krankheitserreger wirkt, wobei dadurch der Raum oder die Räume in kleinere Raumsegmente aufgeteilt werden.

18. Verfahren nach Anspruch 12, ferner umfassend das Detektieren einer Bewegung oder einer Anwesenheit einer oder mehrerer Personen (P) in dem Raum und das automatische Ein- oder Ausschalten einer oder mehrerer Strahlungsquellen (10) abhängig von wenigstens der Anwesenheit der Person (P), und das Abschalten der betreffenden Strahlungsquelle (10), wenn die aus den von der Sensoranordnung detektierten Bewegungsdaten es wahrscheinlich erscheinen lassen, dass eine der Personen (P) das betreffende Strahlungsfeld (10b) durchqueren möchte.
